# EUROPEAN PATENT APPLICATION

(11) **EP 1 038 510 A2**
(43) Date of publication of application: **27.09.2000**
(21) Application number: 00106146.4
(22) Date of filing: 21.03.2000
(51) Int. Cl.: A61F 13/00, A61F 7/00

(54) **Cooling bandage**

(30) Priority: 26.03.1999 IT TO990236
(71) Applicant: Karbix Establishment, 9490 Vaduz (LI)
(72) Inventor: Ferrando, Ugo, 10100 Torino (IT)
(74) Representative: Lotti, Giorgio

(57) **Abstract**

*Bandage (1) provided with a first inner tubular surface (5) and a second outer tubular surface (6) which are coaxial to each other and to a longitudinal axis (A) of development of the bandage (1), a waterproof chamber (3) defined by the first and the second surface (5, 6), and a cooling liquid (L) place inside the chamber (3).*

## Description

The present invention relates to a bandage which is advantageously useful in the medico-surgical field, and more particularly, in the andrological field.

Normally, in the andrological field, once the penis has undergone an operation, two very important activities, both of which are strictly indispensable, are required for an optimal post-operation course: the first activity consists of applying cooling compresses around the penis for a certain period of time, and the second activity consists of binding up the penis with a compressive bandage, which is made of gauze which is firmly wound around the penis in such a way as to press the external surface of the penis with a pressure of a certain value.

Nowadays, the execution of both activities presents some drawbacks. In the first place, the application of said cooling compresses turns out to be particularly irritating for the patient because such compresses are not located in the operated area only, then it turns out particularly laborious to the medical staff because the compresses do not allow a continuous observation of the area which has been operated on. Furthermore, despite the anti-swelling action of the cooling compresses, it is possible that the penis might swell because of the past operation causing the bandage to fit poorly need to be replaced.

At least, as far as a bandage can be well made, and as far as the current compresses can be substantially shaped around a penis, the realisation of a multi-layer wrapping turns out to be very difficult and demanding and means that the operation takes a relatively long time and results in a relatively high waste of materials.

It is an object of the present invention to provide a bandage, which allows the aforementioned activities to be carried out in a simple and economic way, and allows the reduction to a minimum of both the nuisance and discomfort to the patient and the work and effort on the part of the medical staff.

According to a preferred embodiment of the invention, a bandage is realised characterised by the fact that it comprises a first inner tubular surface and a second outer tubular surface which are coaxial to each other and to a longitudinal axis of development of the bandage, a first waterproof chamber defined by the first and the second surface, and a cooling liquid placed inside the firs chamber.

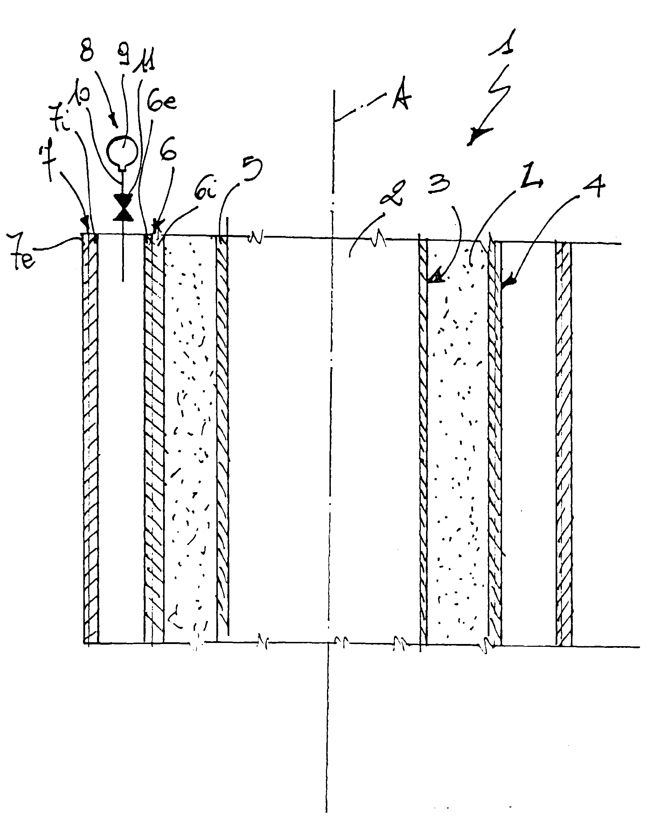

The invention will now be described with reference to the accompanying drawing, which shows, with parts in section and parts removed for the sake of clarity, a preferred and non-limiting embodiment of the bandage given by way of example.

With reference to the accompanying drawing, the number 1 indicates, in its entirety, a cooling compressive bandage, whose compression capacity is capable of being modulated. The bandage is able to be applied on a cylindrical body 2, and is not only able to cool the body 2 for a certain period of time, but is also able to apply a substantial radial pressure on the body 2.

As a matter of fact, the body 2 can be defined by a penis which has just undergone a surgical operation, or by an arm or a leg, and the bandage 1 will have dimensions and shapes such that it can be adapted to the individual mode of use.

The bandage 1 has a longitudinal axis A of development, and comprises a waterproof chamber 3 coaxially placed to the axis A, and a further waterproof chamber 4, which is placed coaxial to and outside the chamber 3, and presents a volume whose capacity can be variable.

The chamber 3 is radially and internally delimited by a first substantially cylindrical wall 5 able to be placed in direct contact with the body 2, and filled by an incompressible cooling liquid L of a known kind. The liquid L is part of the bandage 1, and, after being placed in an environment of very low temperature, it is able to exchange heat with the outside environment in a gradual and prolonged way.

The chamber 4 is radially and internally delimited by a second substantially cylindrical wall 6, which is part of the bandage 1, and in turn comprises an inner portion 6i and an outer portion 6e. Furthermore, the chamber 4 is radially and externally delimited by a third substantially cylindrical wall 7, which forms part of the bandage 1, and is placed coaxial to and outside the wall 6.

The portion 6i and the portion 6e can be distinguished one from the other to delimit, respectively, outwardly the chamber 3 and inwardly the chamber 4, or they can form a single tubular barrel to separate the two chambers 3 and 4: these two described cases are alternatives and are perfectly interchangeable one with the other.

The wall 7 presents a value of the deformation strength greater than a value of the deformation strength of the wall 6, and comprises an inner portion 7i and an outer portion 7e, which can form a single tubular barrel, or can be distinguished one from the other. In this case, the outer portion 7e can be defined by a gauze helicoidally wounded around the portion 7i. As in the case of the wall 6, the last two described cases are alternatives and are perfectly interchangeable one with the other.

For the sake of clarity, we will make reference to the case in which both walls 6 and 7 are defined by relevant single barrels made of a waterproof material having different mechanical characteristics for the wall 6 and for the wall 7 from the point of view of their deformation strength.

The bandage 1 comprises, at the end, a pneumatic device 8 connected to the chamber 4 and able to vary the capacity of the volume of the chamber 4. More particularly, the pneumatic device 8 comprises a pump 9, a connecting pipe 10 between the pump 9 and chamber 4, and a valve 11, which is placed along the pipe 10, and can be operated to modulate, together with the pump 9, the value of the pressure inside the chamber 4, or to make this value equal to the value of the atmospheric pressure.

The bandage 1 is normally kept in a low place with a low temperature so as to cool the cooling liquid L inside the chamber 3, and is put on the body 2: the choice of the proper kind of bandage 1 is made in such a way that the inner diameter of the wall 5 is substantially greater than the outside diameter of the body 2.

Once the bandage 1 has been put on the body 2, the liquid L will start to take away heat from the body 2 lowing its temperature and performing its anti-swelling action, which could turn out to be necessary but not sufficient unless it were also followed by a compressive active action on the body 2. Therefore, before the body 2 can eventually increase in volume and exert a compressive action of the expanding kind against the wall 5 receiving by means of the wall 5 a passive compressive counteraction, the pneumatic device 8 increases the volume of the chamber 4, and, as a consequence, the value of the air pressure inside the chamber 4.

Because of the different mechanical characteristics of the wall 6 and the wall 7, the latter, as a result of the increase of the value of the pressure inside the chamber 4, opposes a deformation strength greater than the deformation strength of the wall 6, which collapses and compresses both the chamber 4 and the body 6 with a substantial radial load displaced all along the axis A.

The application of a Doppler device on the body 2 allows the monitoring of the arterial flow rate of the body 2, and the presence of the pneumatic device 8 allows the variation of the pressure inside the chamber 4 according to this monitoring. The pneumatic device 8 and the chamber 4 can also be excluded in the case in which the anti-swelling action of the liquid L is sufficient. Therefore, in accordance with a not shown embodiment, the bandage 1 is lacking in both the chamber 4 and the pneumatic device 8. In this case, the outer portion 6e of the wall 6 could be made of a gauze firmly wound around the chamber 3.

Finally, in accordance with a further not shown embodiment of the bandage 1, the walls 5, 6 and 7, instead of being annular continuous walls such as the previously described walls above, are annular discontinuous walls, that is they are provided with respective pairs of extremity lips parallel to the axis A, and are able to be laid two by two one upon the other: in this way, the bandage 1 is simply wrapped around the body 2, and results to be easily used in all first aid operations where, for example, a limb has to be immobilised without being moved from its position.

In this case, the outer portion 7e of the wall 7 will be provided with a hooking device, which is substantially distributed along the relevant two lips, and is able to avoid the separation of the lips at the same moment in which the volume of the chamber 4 start increasing.

The adoption of materials which may be sterilised for the bandage 1 means that it can easily be used time after time, even if, bearing in mind its own manufacturing simplicity, the utilisation of disposable bandages is not so disadvantageous from the point of view of running expenses.

While a specific embodiment of the invention has been disclosed, it is to be understood that such disclosure has been merely for the purpose of illustration and that the invention is not to be limited in any manner thereby. Various modifications will be apparent to those skilled in the art in view of the foregoing example. The scope of the invention is to be limited only by the appended claims.

## Claims

1. Bandage (1) characterised by the fact that it comprises a first inner tubular surface (5) and a second outer tubular surface (6) which are coaxial to each other and to a longitudinal axis (A) of development of the bandage (1), a first waterproof chamber (3) defined by the first and the second surface (5, 6), and a cooling liquid (L) placed inside the first chamber (3).

2. Bandage as claimed in claim 1, characterised by the fact that it comprises: a third tubular surface (7) placed coaxial to said axis (A) and radially outside of said second surface (6); a second waterproof chamber (4) defined by the second and the third surface (6, 7) and presenting a variable volume capacity; and pneumatic means (8) linked to the second chamber (4) to vary the capacity of the volume of the second chamber (4).

3. Bandage as claimed in claim 2, characterised by the fact that the third surface (7) presents a value of deformation strength greater than a value of the deformation strength of said second surface (6)

4. Bandage as claimed in claim 2 or 3, characterised by the fact that said pneumatic means (8) comprise a control valve (11) to modulate the variation of the capacity of said volume.

5. Bandage as claimed in claim 1 or 2, characterised by the fact that the second surface (6) is defined by a first tubular barrel.

6. Bandage as claimed in claim 1, 2 or 5, characterised by the fact that the third surface (7) is defined by a second tubular barrel.
